# FASCICULE DE BREVET EUROPEEN

(11) **EP 1 541 156 B1**
(45) Date de publication et mention de la délivrance du brevet: **31.12.2008**
(21) Numéro de dépôt: 04300830.9
(22) Date de dépôt: 01.12.2004
(51) Int. Cl.: A61K 33/00, A61P 25/00

(54) **Médicament gazeux inhalable à base d'argon pour le traitement des neuro-intoxications**
Argon enthaltendes Arzneimittel zum Inhalieren und dessen Verwendung zur Behandlung von Neurotoxizität
Inhalation medicament comprising argon and its use for the treatment of neurotoxicity

(30) Priorité: 08.12.2003 FR 0350997
(43) Date de publication de la demande: 15.06.2005
(60) Demande divisionnaire: 08165103.6
(73) Titulaire: Air Liquide Santé (International), 75341 Paris Cedex 07 (FR)
(72) Inventeur: Lemaire, Marc, 75014 Paris (FR); Abraini, Jacques, 14000 Caen (FR)
(74) Mandataire: Pittis, Olivier

(56) Documents cités:
- WO-A-97/34870
- US-A- 584 556
- ABRAINI JACQUES H ET AL: "Gamma-aminobutyric acid neuropharmacological investigations on narcosis produced by nitrogen, argon, or nitrous oxide." ANESTHESIA AND ANALGESIA. MAR 2003, vol. 96, no. 3, mars 2003 (2003-03), pages 746-749 , tab, XP008033053 ISSN: 0003-2999
- CUMMING G ET AL: "INHALED ARGON BOLUSES IN MAN" JOURNAL OF APPLIED PHYSIOLOGY, vol. 27, no. 4, 1969, pages 447-451, XP008033052 ISSN: 0021-8987

## Description

L'invention porte sur l'utilisation d'argon pour fabriquer tout ou partie d'un médicament inhalable destiné à traiter ou à prévenir une pathologie à effet neurotoxique, c'est-à-dire une neuro-intoxication.

Dans les pathologies liées aux effets neurotoxiques des drogues générant une addiction, telles les amphétamines, il est admis que la neurotransmission dopaminergique d'origine nigrostriatale et mésolimbique participe des effets psycho-stimulants et neurotoxiques de ces drogues.

Cependant, des travaux récents de Del Arco et al., Neuropharmacology, 1999, vol. 38, p. 943-954, ont montré que les effets facilitateurs des amphétamines ne se limitent pas à la neurotransmission dopaminergique.

Ainsi, au niveau du complexe striatum-noyau accumbens, les amphétamines induisent non seulement une augmentation de la libération de dopamine mais perturbe les systèmes de neurotransmission à la sérotonine, à la taurine, à l'acide γ-amino-butyrique (GABA), à la noradrénaline et au glutamate.

De façon particulièrement intéressante, il a été montré que l'inhibition spécifique des transporteurs du glutamate permet de diminuer à la fois l'hyperactivité (David et al. Neuropharmacology, 2001, vol. p. 409-411) et l'augmentation de glutamate, mais pas de dopamine (Del Arco et al., Neuropharmacology, 1999, vol. 38, p. 943-954), consécutives à l'injection d'amphétamines, suggérant ainsi un rôle déterminant du glutamate dans tes effets psychostimulants des amphétamines.

Par ailleurs, des travaux récents, réalisés in vitro, ont montré que le xénon et le protoxyde d'azote (N₂O) peuvent se comporter comme des antagonistes des récepteurs glutamatergiques au N-Méthyl-D-Aspartate (NMDA) ; Franks et al., Nature, 1998, vol. 396, p. 324 ; Jevtovic-Todorovic et al., Nature Med., 1998, vol. 4, p. 460- 463.

En outre, dans le cadre de l'étude du système opioïde hyperalgésique endogène dans la réponse placebo négative, F.J. Lichtigfeld et M.A. Gillman, Intem. J. Neuroscience, 1989, vol. 49, p. 71-74 concluent à un effet du protoxyde d'azote sur le sevrage alcoolique un peu meilleur que l'effet placebo, bien que, pour plus de 50% des individus, un effet positif identique a aussi été constaté avec le placebo.

Toutefois, les mêmes auteurs ajoutent, dans Nitrous Oxide and the Aws, p. 785, que l'effet bénéfique du protoxyde d'azote dépendant étroitement de sa concentration car des concentrations anesthésiques ou pré-anesthésiques sont inefficaces, voire même contre-productives dans certains cas ; une concentration analgésique étant recommandée.

Dans Postgrad. Med. J, Clinical Toxicology, 1990, vol. 66, p. 543-546, les mêmes auteurs expliquent que les concentrations en protoxyde d'azote peuvent varier de moins de 15% à plus de 70% selon les individus et ce, en fonction de leur degré de dépendance à l'alcool.

Par ailleurs, le document EP-A-1158992 enseigne l'utilisation de xénon ou d'un mélange de xénon avec de l'oxygène, de l'azote ou de l'air pour traiter les neuro-intoxications.

Toutefois, utilisation de xénon ou des mélanges décrits par ce document n'est pas totalement satisfaisante en pratique, notamment du fait de l'apparition d'une toxicité pour certaines teneurs en xénon et compte tenu du coût élevé de ce composé ; David et al.; J. Cereb. Blood Flow. Metab., 2003, vol. 23, p.1168-1173.

Le document WO 97/34870 propose l'utilisation de pyrroalécarboxamides en tant que ligands des récepteurs GABA cérébraux, utilisables pour le traitement de l'anxiété, des troubles du sommeil, et des overdoses engendrées par les drogues à base de benzodiazépine ; et pour l'amélioration de la mémoire.

Par ailleurs, le document « Gamma-Aminobutyric Acid Neuropharmacological Investigations on Narcosis Produced by Nitrogen, Argon, or Nitrous Oxide » de J. Abraini et al. Anesth. Analg. 2003, 96:746-9, p.746 à 749, enseigne que l'azote et l'argon, mais pas le protoxyde d'azote, peuvent agir directement en potentialisant la neurotransmission GABA au niveau des récepteurs GABA_{A}. Ceci permet d'expliquer certains mécanismes de l'action narcotique de l'azote et de l'argon qui pourraient être similaires à ceux des anesthésiques inhalés.

Enfin le brevet US-A-5,846,556 propose une composition à inhaler contenant de l'azote, de l'oxygène, un gaz inerte, tels l'argon, le krypton, le néon, l'hélium ou le xénon, du dioxyde de carbone et un composé anesthésique. Cette composition est préconisée pour les personnes souhaitant arrêter de fumer, en plongée profonde, subséquemment à un trajet en avion ou lors de la naissance pour induire une relaxation.

La présente invention s'inscrit dans ce contexte et vise à améliorer les médicaments inhalables existant destinés à prévenir ou traiter efficacement une neuro-intoxication chez l'homme, laquelle se caractérise par un dysfonctionnement cérébral d'un ou de plusieurs systèmes de neurotransmission.

La solution de l'invention porte alors sur l'utilisation d'argon (Ar) gazeux pour fabriquer tout ou partie d'un médicament inhalable destiné à prévenir ou à traiter une neuro-intoxication chez l'homme, c'est-à-dire sur de l'argon gazeux pour une utilisation en tant que médicament inhalabe destiné à prévenir ou traiter une telle neuro-intoxication.

Par neuro-intoxication, on entend un trouble, un désordre ou une pathologie du système nerveux central dont l'étiopathogénie implique, au moins pour partie, un processus excito-toxique, notamment un dysfonctionnement de la neurotransmission excitatrice au glutamate ; voir notamment le document Parsons et al., Drug News Perspect., 1998, vol.11, pages 523-569.

En conséquence, entre dans le cadre de la présente invention le traitement notamment:
- d es effets neurotoxiques de drogues ou autres substances pouvant générer une addiction comme les amphétamines et dérivés amphétaminiques, les substances opiacées et leurs dérivés, la cocaine et ses dérivés, le tabac, le cannabis et/ou l'alcool ;
- des accidents cérébraux aigus comme les traumas crâniens et les accidents vasculaires cérébraux (AVC), y compris l'ischémie cérébrale ;
- des maladies neuro-dégénératives comme la maladie d'Alzheimer, la maladie de Parkinson, la maladie (chorée) de Huntington, la sclérose latérale amyotrophique, l'encépholomyélite aiguë disséminée, la dyskinésie tardive, et les dégénéresences olivopontocérébelleuses ; et
- diverses pathologies psychiatriques ou neurologiques comme les troubles anxieux, les troubles psychotiques, notamment la schizophrénie et l'épilepsie sous ses diverses formes.

Selon le cas, l'utilisation d'argon selon l'invention peut comprendre l'une ou plusieurs des caractéristiques techniques suivantes:
- la neuro-intoxication résulte d'un dysfonctionnement cérébral, à savoir un excès ou une diminution, d'un ou plusieurs systèmes de neurotransmetteurs.
- le mélange contenant de l'argon en une proportion efficace agit sur au moins un récepteur cérébral afin de réguler le fonctionnement des systèmes de neurotransmission à la dopamine, au glutamate, à la sérotonine, à la taurine, à l'acétylcholine, au GABA et/ou à la noradrénaline.
- la proportion volumique d'argon dans ledit mélange gazeux est comprise entre 15 et 80%.
- la proportion volumique d'argon est comprise entre 30 et 75%.
- l'argon est en mélange gazeux avec au moins un composé gazeux additionnel choisi parmi le xénon, le krypton et le protoxyde d'azote (N₂O).
- le médicament gazeux contient, en outre, de l'oxygène, de l'azote ou leur mélanges, en particulier de l'air.
- le mélange gazeux est un mélange binaire constitué d'argon et d'oxygène pour le reste, de préférence le médicament gazeux est prêt à l'emploi, c'est-à-dire qu'il peut être administré au patient directement sans subir de pré-dilution.
- la neuro-intoxication est choisie parmi les troubles, désordres ou pathologies du système nerveux central dont l'étiopathogènie implique, au moins pour partie, un processus excititoxique, comme les effets neurotoxiques de drogues ou substances pouvant générer un état d'addiction, les accidents cérébraux aigus, les maladies neuro-dégénératives, et diverses pathologies psychiatriques ou neurologiques. Par "neuro-intoxication engendrant un état d'addiction", on entend un trouble, un désordre ou une pathologie lié aux effets neurotoxiques d'une drogue, molécule ou substance générant une addiction ou une accoutumance chez l'homme ou l'animal. La substance, drogue ou molécule générant l'addiction est choisie dans le groupe formé par les amphétamines et leurs dérivés, les substances opiacées et leurs dérivés, la cocaïne et ses dérivés, le tabac, l'alcool et le cannabis, ou toute autre drogue similaire ou analogue. Par "accident cérébral aigu", on entend un trouble, un désordre ou une pathologie consécutif à un événement brutal et soudain d'origine exogène ou endogène. L'évènement exogène peut être un trauma crânien, alors que l'évènement endogène peut être la rupture ou l'occlusion d'une artère ou d'un vaisseau sanguin cérébral. Par maladie neuro-dégénératives, on entend un trouble, un désordre ou une pathologie lié à la dégénérescence et la mort de certains neurones cérébraux.
- le médicament inhalable est conditionné à une pression de 2 bars à 350 bars, de préférence entre 2 bars et 200 bars.
- le mélange gazeux est formé d'argon et d'oxygène.
- le mélange gazeux est formé de 20 à 80 % en volume d'argon et d'oxygène pour le reste, de préférence de 30 à 75% d'argon. Dans tous les cas, les proportions d'argon et/ou d'oxygène dans le mélange gazeux pourront être ajustées en fonction de la durée du traitement.

Autrement dit, l'idée à la base de la présente invention est donc que les propriétés agonistes des récepteurs de type A de la neurotransmission inhibitrice au GABA de l'argon (voir Abraini et al., Anesth Analg, 2003, vol. 96, p. 746-749, 2003) peuvent être utilisées, de par leur caractère inhibiteur, pour limiter les effets excitateurs du glutamate afin de prévenir et/ou de traiter les neuro-intoxications, notamment les effets neurotoxiques de drogues ou substances générant une addiction, telles que les amphétamines et leurs dérivés, les substances opiacées et leurs dérivés, la cocaïne et ses dérivés, le tabac, l'alcool, le cannabis ou tout autre substance engendrant une dépendance.

De façon générale, le médicament gazeux selon invention est administrable au patient par ses voies aériennes supérieures, c'est-à-dire par inhalation via son nez et/ou sa bouche, au moyen d'un dispositif d'administration adapté comprenant une interface respiratoire patient, tel qu'un masque respiratoire ou une sonde trachéale, une ou plusieurs canalisations d'alimentation servant à acheminer le médicament gazeux depuis une source contenant ledit médicament jusqu'à l'interface, et un ventilateur médical servant à envoyer et/ou extraire le gaz du patient.

Exemple : Evaluation du potentiel neuroprotecteur de l'argon gazeux, administré seul ou en mélange avec du protoxyde d'azote, sur le développement et l'expression de la sensibilisation à la D-amphétamine.

L'objectif de l'étude est d'évaluer le potentiel neuroprotecteur sur la sensibilisation à la D-amphétamine de l'argon dont les mécanismes d'action encore mal connus pourraient transiter par une action agoniste des récepteurs GABA_{A}, notamment du site aux benzodiazépines, l'argon étant administré seul ou en mélange ; Abraini et al., Anesth Analg, 2003, vol. 96, p. 746-749, 2003.

Pour ce faire, on a utilisé des rats mâles adultes Sprague-Dawley d'un poids d'environ 220 g à leur arrivée au laboratoire. Pendant toute la durée de l'étude, les animaux ont été placés dans des conditions d'animalerie standard par groupe de 8, de façon à éviter l'apparition d'une réaction de stress consécutive à l'isolement. Ils disposaient d'eau et de nourriture *ad libitum*.

Le protocole de sensibilisation à la d-amphétamine suivi et les essais de traitement par administration de gaz mis en oeuvre ont été les suivants.

Durant 3 jours consécutifs (de J1 à J3), 8 groupes d'animaux (8 rats par groupe) ont reçu par voie intrapéritonéale (i.p.), soit de la d-amphétamine (amph : 1mg/ml/kg), soit une solution saline (saline :1 ml/kg) pour les animaux témoins.

Après chaque injection de d-amphétamine, les rats étaient immédiatement placés pendant 3 heures dans une enceinte close, d'un volume de 100 litres, balayée en régime dynamique, soit :
- par de l'air (Groupe 1 : saline ; Groupe 2 : amph).
- par un mélange d'argon à 37.5 vol.% et de protoxyde d'azote à 37.5 vol.% (Groupe 3 : saline ; Groupe 4 : amph), le complément étant de l'oxygène.
- par un mélange d'argon à 50 vol.% et de protoxyde d'azote à 25 vol.% (Groupe 5 : saline ; Groupe 6 : amph), le complément étant de l'oxygène.
- par de l'argon à 75 vol.% (Groupe 7 : saline ; Groupe 8 : amph), le complément étant de l'oxygène.

Les gaz utilisés dans cette étude ont été administrés en régime dynamique à une vitesse initiale de 10 I.min⁻¹ pendant 30 minutes, puis à une vitesse constante de 1 I.min⁻¹ pendant 2 h 30.

En procédant ainsi, la concentration effective après 30 minutes de traitement est égale à 95 % de la concentration finale désirée (correspondant au mélange utilisé) et la valeur dose x temps cumulée est supérieure de plus de 25 % à la valeur dose x temps obtenue en utilisant comme précédemment une vitesse d'admission constante des gaz de 5 I.min⁻¹; voir le document Abraini et David, pour Air Liquide Santé International « étude du potentiel neuroprotecteur du xénon et du protoxyde d'azote » Mai 2001-Octobre 2003), ce qui permet d'optimiser le traitement dans sa phase initiale ; es valeurs dose x temps cumulées totales sont sensiblement égales ; voir Tableau 1 ci-après.

**Tableau 1**

| 1 - % [finale] | Kc | Tps (100/10) | D*T | D*T cumulé | Tps (100/5) | D*T | D*T cumulé |
|---|---|---|---|---|---|---|---|
| 1 | | | | | 0 | | |
| 0,95 | 0,051 | 0,5 | 0,6 | 0,6 | 1,0 | 1,28 | 1,28 |
| 0,9 | 0,105 | 1,1 | 2,0 | 2,6 | 2,1 | 4,06 | 5,34 |
| 0,85 | 0,163 | 1,6 | 3,6 | 6,2 | 3,3 | 7,14 | 12,48 |
| 0,8 | 0,223 | 2,2 | 5,3 | 11,5 | 4,5 | 10,61 | 23,09 |
| 0,75 | 0,288 | 2,9 | 7,3 | 18,8 | 5,8 | 14,52 | 37,61 |
| 0,7 | 0,357 | 3,6 | 9,5 | 28,3 | 7,1 | 18,97 | 56,58 |
| 0,65 | 0,431 | 4,3 | 12,0 | 40,3 | 8,6 | 24,09 | 80,67 |
| 0,6 | 0,511 | 5,1 | 15,0 | 55,3 | 10,2 | 30,02 | 110,68 |
| 0,55 | 0,598 | 6,0 | 18,5 | 73,8 | 12,0 | 36,98 | 147,66 |
| 0,5 | 0,693 | 6,9 | 22,6 | 96,4 | 13,9 | 45,27 | 192,94 |
| 0,45 | 0,799 | 8,0 | 27,7 | 124,1 | 16,0 | 55,31 | 248,25 |
| 0,4 | 0,916 | 9,2 | 33,9 | 157,9 | 18,3 | 67,73 | 315,98 |
| 0,35 | 1,050 | 10,5 | 41,7 | 199,7 | 21,0 | 83,46 | 399,43 |
| 0,3 | 1,204 | 12,0 | 52,0 | 251,7 | 24,1 | 104,05 | 503,48 |
| 0,25 | 1,386 | 13,9 | 66,1 | 317,8 | 27,7 | 132,18 | 635,67 |
| 0,2 | 1,609 | 16,1 | 86,5 | 404,3 | 32,2 | 172,94 | 808,60 |
| 0,15 | 1,897 | 19,0 | 118,7 | 522,9 | 37,9 | 237,34 | 1045,94 |
| 0,1 | 2,303 | 23,0 | 177,4 | 700,3 | 46,1 | 354,78 | 1400,72 |
| 0,05 | 2,996 | 30,0 | 320,6 | 1020,9 | 59,9 | 641,16 | 2041,89 |
| 0,01 | 4,605 | 160,9 | 6352,8 | 7373,7 | 92,1 | 1561,15 | 3603,04 |
| 0,01 | 4,605 | 180 | 943,3 | 8317,0 | 180 | 4350,88 | 7953,92 |

Le Tableau 1 indique les doses cumulées (dose x temps ; D * T cumulé) en fonction du régime de balayage dynamique utilisé (correspondant à la vitesse d'admission des gaz ou mélanges de gaz) pour saturer une enceinte d'un volume de 100 litres. On remarque qu'au bout de 30 minutes, la dose cumulée obtenue en utilisant une vitesse initiale de 10 l.min⁻¹ (suivie d'une vitesse constante de 1 I.min⁻¹ pendant 2 h 30) est supérieure d'environ 25 % à la dose cumulée obtenue en utilisant une vitesse constante de 5 I.min⁻¹.

L'activité locomotrice et l'activité de redressement des animaux ont été évaluées à J6, après une injection i.p. d'une solution saline (1 ml/kg) afin de déterminer les effets propres des traitements administrés avec les différents gaz et mélanges de gaz, et à J7 après une administration i.p. de d-amphétamine (1/mg/ml/kg) afin d'évaluer les effets des gaz et mélanges de gaz sur la sensibilisation à la d-amphétamine.

L'activité locomotrice et l'activité stéréotypique de redressement des animaux en réponse à ces injections ont été enregistrées au moyen d'un système d'actimétrie à cellules photoélectriques (Imétronic, Pessac, France).

La d-amphétamine (d-amphétamine sulfate ref. A5880) a été acquise auprès de Sigma-Aldrich (Illkirch, France).

L'air médical, l'argon à 75 vol.%, et le mélange de protoxyde d'azote à 50 vol.% et d'argon à 75 vol.%, le complément étant de l'oxygène, ont été fournis par Air Liquide Santé International (Paris, France).

Le mélange de protoxyde d'azote à 37.5 vol.% et d'argon à 37.5 vol.%, le complément étant de l'oxygène, a été réalisé à partir de protoxyde d'azote à 75 vol.% et d'argon à 75 vol.%, fournis par Air Liquide Santé International, à l'aide de débit-litres calibrés également fournis par Air Liquide Santé International.

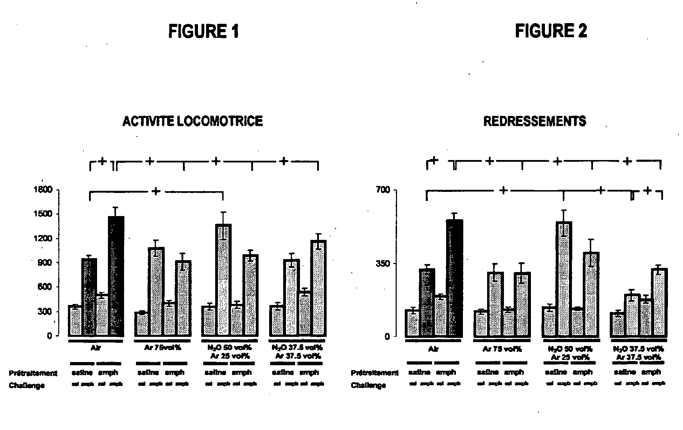

Les résultats obtenus (voir Figures 1 et 2) sont exprimés par la moyenne ± l'erreur standard à la moyenne. La comparaison des groupes a été réalisée au moyen de tests non-paramétriques : l'analyse de variance de Kruskall-Wallis, complétée en cas de résultat significatif par le test U de Mann-Whitney.

Les parties gauches des Figures 1 et 2 illustrent le processus de sensibilisation induit par l'administration répétée de d-amphétamine.

Plus précisément, la Figure 1 illustre les effets, à J7, sur l'activité locomotrice induit par l'injection répétée de d-amphétamine, alors que la Figure 2 illustre la production de mouvements stéréotypiques, *i*.*e*. les redressements, induit par l'administration répétée de d-amphétamine (1 mg/kg).

Le challenge à la d-amphétamine engendre une augmentation de l'activité locomotrice ainsi que des mouvements stéréotypiques, de sorte que l'activité locomotrice et les mouvements stéréotypiques (c'est-à-dire les redressements) des animaux prétraités à la d-amphetamine apparaissent significativement supérieurs à ceux des rats témoins prétraités au moyen d'une solution saline, lors du test à la d-amphétamine réalisé à J7 (P < 0.05).

Les Figures 1 et 2 illustrent les effets sur l'activité locomotrice et les redressements induits par l'administration répétée de d-amphétamine d'un traitement au moyen d'argon à 75 vol.%, d'un mélange de protoxyde d'azote à 50 vol.% et d'argon à 25 vol.%, ou d'un mélange de protoxyde d'azote à 37.5 vol.% et d'argon à 37.5 vol.% (le complément étant de l'oxygène).

L'exposition, immédiatement après administration de d-amphétamine, à l'argon à 75 vol.% ou à un mélange de protoxyde d'azote à 50 vol.% et d'argon à 25 vol.% ou à un mélange de protoxyde d'azote à 37.5 vol.% et d'argon à 37.5 vol.% induit un blocage du développement de l'activité locomotrice correspondant au processus de sensibilisation à la d-amphétamine.

L'activité locomotrice obtenue à J7 lors du challenge à la d-amphétamine chez les rats sensibilisés pendant 3 jours à la d-amphétamine et traités avec (i) de l'argon à 75 vol.% ou (ii) un mélange de protoxyde d'azote à 50 vol.% et d'argon à 25 vol.% ou (iii) un mélange de protoxyde d'azote à 37.5 vol.% et d'argon à 37.5 vol.%, est inférieure à celle des rats sensibilisés à la d-amphétamine et traités à l'air, mais non significativement différente de l'activité locomotrice des animaux ayant reçus, pendant 3 jours, une solution saline et (i) de l'argon à 75 vol.% ou (ii) un mélange de protoxyde d'azote à 50 vol.% et d'argon à 25 vol.% ou (iii) un mélange de protoxyde d'azote à 37.5 vol.% et d'argon à 37.5 vol.% (correspondant à une injection aiguë de d-amphétamine).

Ces résultats montrent un effet inhibiteur total de l'argon à 75 vol.% (P<0.005), du mélange de protoxyde d'azote à 50 vol.% et d'argon à 25 vol.% (P<0.002), et de façon moindre du mélange de protoxyde d'azote à 37.5 vol.% et d'argon à 37.5 vol.% (P<0.05) sur l'hyperactivité locomotrice inhérente au développement de la sensibilisation à la d-amphétamine.

Cependant, il convient de noter chez les rats ayant reçus pendant 3 jours une solution saline et exposés au mélange de protoxyde d'azote à 50 vol% et d'argon à 25 vol% que l'activité locomotrice engendrée par le challenge à la d-amphétamine, réalisé à J7, est significativement supérieure (P<0.01) à activité locomotrice mesurée lors du même challenge chez les animaux ayant reçu pendant 3 jours une solution saline et exposés à l'air ; le niveau d'activité locomotrice atteint étant comparable à l'activité locomotrice généralement enregistrée après sensibilisation.

Ce résultat pourrait témoigner d'un effet potentiellement neurotoxique du mélange de protoxyde d'azote à 50 vol.% et d'argon à 25 vol.%, semblable aux effets déjà connus pour le xénon à 75 vol.% ; voir Abraini et David, pour Air Liquide Santé International « Etude du potentiel neuroprotecteur du xénon et du protoxyde d'azote » Mai 2001-Octobre 2003*.*

Par ailleurs, l'exposition à l'argon à 75 vol.%, au mélange de protoxyde d'azote à 50 vol.% et d'argon à 25 vol.% ou au mélange de protoxyde d'azote à 37.5 vol.% et d'argon à 37.5 vol.%, immédiatement après injection de d-amphétamine, induit un blocage du développement de l'activité de redressement inhérente au processus de sensibilisation à la d-amphétamine.

L'activité stéréotypique de redressement obtenue à J7 lors du challenge à la d-amphétamine chez les rats sensibilisés pendant 3 jours à la d-amphétamine et traités avec (i) de l'argon à 75 vol % ou (ii) un mélange de protoxyde d'azote à 50 vol.% et d'argon à 25 vol.% ou (iii) un mélange de protoxyde d'azote à 37.5 vol.% et d'argon à 37.5 vol.%, est inférieure à celle des rats sensibilisés pendant 3 jours à la d-amphétamine et traités à l'air, mais non significativement différente de l'activité Ide redressement des animaux ayant reçus pendant 3 jours une solution saline et (i) de l'argon à 75 vol.% ou (ii) un mélange de protoxyde d'azote à 50 vol.% et d'argon à 25 vol.% ou (iii) un mélange de protoxyde d'azote à 37.5 vol.% et d'argon à 37.5 vol.% (correspondant à une injection aiguë de d-amphétamine).

Ces résultats témoignent d'un effet inhibiteur de l'argon à 75 vol.% (P<0.005), du mélange de protoxyde d'azote à 37.5 vol.% et d'argon à 37.5 vol.% (P<0.001) et de façon moindre du mélange de protoxyde d'azote à 50 vol.% et d'argon à 25 vol.% (P<0.02) sur l'activité de redressement correspondant au développement de la sensibilisation à la d-amphétamine.

Cependant, il convient là aussi de noter chez les rats ayant recus pendant 3 jours une solution saline et exposés au mélange de protoxyde d'azote à 50 vol.% et d'argon à 25 vol.% que l'activité de redressement engendrée par le challenge à la d-amphétamine réalisé à J7 apparaît significativement supérieure (P<0.002) à l'activité de redressement mesurée lors du même challenge chez les animaux ayant reçus pendant 3 jours une solution saline et exposés à l'air ; le niveau d'activité stéréotypique atteint étant comparable à l'activité de redressement généralement enregistrée après sensibilisation.

Ce résultat, qui corroborent les données obtenues ci-avant pour l'activité locomotrice, renforcent l'idée d'un effet potentiellement neurotoxique, du mélange de protoxyde d'azote à 50 vol.% et d'argon à 25 vol.%.

En définitive, bien que le mélange de protoxyde d'azote à 50 vol.% et d'argon à 25 vol.% montre un effet inhibiteur sur l'activité locomotrice et l'activité de redressement induites par l'administration répétée de d-amphétamine, les résultats obtenus suggèrent un possible effet neurotoxique de ce mélange.

Il apparaît donc que les autres gaz et mélanges gazeux testés, à savoir l'argon à 75 vol.% et le mélange, de protoxyde d'azote à 37.5 vol.% et d'argon à 37.5 vol.%, sont préférés dans le cadre de la présente invention car ils permettent incontestablement de bloquer à la fois l'hyperactivité locomotrice et l'activité stéréotypique de redressement inhérentes au développement de la sensibilisation à la d-amphétamine et ce, sans qu'il n'ait été observé un quelconque effet neurotoxique.

Autrement dit, l'ensemble des résultats obtenus montre que l'argon, éventuellement additionné de protoxyde d'azote, possède des effets inhibiteurs sur le développement de la sensibilisation à la d-amphétamine.

Plus précisément, concernant l'argon, il convient de souligner de façon particulièrement intéressante, au-delà des données quantitatives qui témoignent que ce gaz possède un effet inhibiteur indéniable à 75 vol.%, que les résultats obtenus d'un point de vue qualitatif sont apparus très surprenants.

Ainsi, dès le 2ème jour de traitement, soit après un jour de sensibilisation à la d-amphétamine et de traitement à l'argon à 75 vol.%, les animaux se sont montrés étonnamment calmes et "coopératifs" pendant toute la suite du protocole expérimental, y compris pendant les injections intrapéritonéales de d-amphétamine.

Cette donnée subjective, mais néanmoins d'un grand intérêt, pourrait témoigner d'un mécanisme d'action radicalement différent de celui du protoxyde d'azote dont les propriétés antagonistes des récepteurs glutamatergiques de type NMDA sont actuellement bien identifiées ; voir notamment *Jevtovic-Todorovic et al*., et *1998 ; Yamakura et al*., *2000.*

Le mode d'action de l'argon demeure encore très largement méconnu, malgré une étude de neuropharmacologie récente, qui a suggéré que l'argon pourrait présenter des propriétés agonistes des récepteurs GABA_{A}; Abraini et al., Anesth Analg, 2003, vol. 96, p. 746-749 2003.

Par ailleurs, l'association de protoxyde d'azote à 37.5 vol.% et d'argon à 37.5 vol.% montre également un effet inhibiteur sur le développement du processus de sensibilisation à la d-amphétamine.

Cet effet obtenu avec des pourcentages de protoxyde d'azote et d'argon respectivement peu élevés pourrait indiquer que ces deux gaz ont des propriétés additives ou synergiques.

Dans ce sens, il faut noter que l'association de protoxyde d'azote à 50 vol.% et d'argon à 25 vol.% pourrait présenter des propriétés potentiellement neurotoxiques et de ce fait, une utilisation en association de protoxyde d'azote et d'argon doit être faite avec précaution, c'est-à-dire en choisissant avec soin les proportions respectives de ces composés.

L'ensemble de ces résultats permet d'envisager un intérêt thérapeutique potentiel de l'argon, seul ou en mélange avec du protoxyde d'azote (N₂O), notamment pour le traitement de l'addiction aux substances psychostimulantes ou pour le moins aux amphétamines.

## Revendications

1. Argon (Ar) gazeux pour une utilisation en tant que médicament inhalable destiné à prévenir ou à traiter une neuro-intoxication chez l'homme.

2. Argon gazeux selon la revendication 1, **caractérisée en ce que** la neuro-intoxication résulte d'un dysfonctionnement cérébral d'un ou plusieurs systèmes à neurotransmetteurs.

3. Argon gazeux selon l'une des revendications 1 ou 2, **caractérisée en ce que** le médicament gazeux contenant de l'argon en une proportion efficace agit sur au moins un récepteur cérébral afin de réguler le fonctionnement des systèmes de neurotransmission à la dopamine, glutamate, sérotonine, acétylcholine, taurine, GABA et/ou noradrénaline.

4. Argon gazeux selon l'une des revendications 1 à 3, **caractérisée en ce que** la proportion volumique d'argon dans ledit médicament gazeux est comprise entre 15 et 80 %.

5. Argon gazeux selon l'une des revendications 1 à 4, **caractérisée en ce que** la proportion volumique d'argon est comprise entre 30 et 75%.

6. Argon gazeux selon l'une des revendications 1 à 5, **caractérisée en ce que** l'argon est en mélange gazeux avec au moins un composé gazeux additionnel choisi parmi le xénon, le krypton et le protoxyde d'azote (N₂O).

7. Argon gazeux selon l'une des revendications 1 à 6, **caractérisée en ce que** le médicament gazeux contient, en outre, de l'oxygène, de l'azote ou leur mélanges, en particulier de l'air.

8. Argon gazeux selon l'une des revendications 1 à 7, **caractérisée en ce que** le médicament gazeux est un mélange gazeux binaire constitué d'argon et d'oxygène pour le reste ou un mélange ternaire constitué d'argon, d'azote et d'oxygène.

9. Argon gazeux selon l'une des revendications 1 à 8, **caractérisée en ce que** la neuro-intoxication est choisie parmi les excito-toxicités engendrant un état d'addiction, les accidents cérébraux aigus, les maladies neuro-dégénératives, et les pathologies psychiatriques ou neurologiques, en particulier les troubles anxieux, les troubles psychotiques, notamment la schizophrénie, et l'épilepsie sous ses diverses formes.

10. Argon gazeux selon l'une des revendications 6 ou 8, **caractérisé en ce que** le médicament gazeux est prêt à l'emploi.

## Claims

1. Use of argon (Ar) gas for producing all or part of an inhalable medicinal product intended to prevent or treat a neurointoxication in humans.

2. Use according to Claim 1, **characterized in that** the neurointoxication results from a cerebral dysfunction of one or more neurotransmitter systems.

3. Use according to either of Claims 1 and 2, **characterized in that** the gaseous medicinal product containing argon in an effective proportion acts on at least one brain receptor in order to regulate the functioning of dopamine-, glutamate-, serotonin-, acetylcholine-, taurine-, GABA- and/or noradrenalin-mediated neurotransmission systems.

4. Use according to one of Claims 1 to 3, **characterized in that** the proportion by volume of argon in said gaseous medicinal product is between 15 and 80%.

5. Use according to one of Claims 1 to 4, **characterized in that** the proportion by volume of argon is between 30 and 75%.

6. Use according to one of Claims 1 to 5, **characterized in that** the argon is in a gaseous mixture with at least one additional gaseous compound chosen from xenon, krypton and nitrous oxide (N₂O).

7. Use according to one of Claims 1 to 6, **characterized in that** the gaseous medicinal product also contains oxygen, nitrogen or mixtures thereof, in particular air.

8. Use according to one of Claims 1 to 7, **characterized in that** the gaseous medicinal product is a binary gaseous mixture consisting of argon and of oxygen for the remainder, or a ternary mixture consisting of argon, of nitrogen and of oxygen.

9. Use according to one of Claims 1 to 8, **characterized in that** the neurointoxication is chosen from excitotoxicities engendering a state of addiction, acute cerebral accidents, neurodegenerative diseases, and psychiatric or neurological pathologies, in particular anxiety conditions, psychotic conditions, in particular schizophrenia, and epilepsy in its various forms.

10. Use according to either of Claims 6 and 8, **characterized in that** the gaseous medicinal product is ready to use.

## Patentansprüche

1. Verwendung von gasförmigem Argon (Ar) für die Herstellung eines inhalierbaren Arzneimittels insgesamt oder eines Teils davon für die Vorbeugung oder Behandlung von Nervenintoxikation beim Menschen.

2. Verwendung nach Anspruch 1, **dadurch gekennzeichnet, daß** die Nervenintoxikation durch Hirndysfunktion von einem oder mehreren Neurotransmittersystemen bedingt ist.

3. Verwendung nach einem der Ansprüche 1 oder 2, **dadurch gekennzeichnet, daß** das gasförmige Arzneimittel Argon in einer auf mindestens einen Hirnrezeptor zur Regulation der Funktion von Neurotransmissionssystemen mit Dopamin, Glutamat, Serotonin, Acetylcholin, Taurin, GABA und/oder Noradrenalin wirksamen Menge enthält.

4. Verwendung nach einem der Ansprüche 1 oder 3, **dadurch gekennzeichnet, daß** die Volumenmenge an Argon in dem gasförmigen Arzneimittel zwischen 15 und 80% beträgt.

5. Verwendung nach einem der Ansprüche 1 oder 4, **dadurch gekennzeichnet, daß** die Volumenmenge an Argon zwischen 30 und 75% beträgt.

6. Verwendung nach einem der Ansprüche 1 bis 5, **dadurch gekennzeichnet, daß** das Argon in einer gasförmigen Mischung mit mindestens einer weiteren gasförmigen Verbindung aus der Reihe Xenon, Krypton und Stickstoffprotoxid (N₂O) vorliegt.

7. Verwendung nach einem der Ansprüche 1 bis 6, **dadurch gekennzeichnet, daß** das gasförmige Arzneimittel weiterhin Sauerstoff, Stickstoff oder Mischungen davon, insbesondere Luft, enthält.

8. Verwendung nach einem der Ansprüche 1 bis 7, **dadurch gekennzeichnet, daß** es sich bei dem gasförmigen Arzneimittel um eine gasförmige Zweiermischung, die aus Argon und als Restkomponente Sauerstoff besteht, oder um eine Dreiermischung, die aus Argon, Stickstoff und Sauerstoff besteht, handelt.

9. Verwendung nach einem der Ansprüche 1 bis 8, **dadurch gekennzeichnet, daß** die Neurointoxikation aus der Reihe Excitotoxizitätserscheinungen, die zu einem Abhängigkeitszustand führen, akute Zerebraltraumen, neurodegenerative Erkrankungen, und psychiatrische oder neurologische Krankheiten, insbesondere Angstzustände, psychotische Zustände, ganz besonders Schizophrenie, sowie Epilepsie in ihren verschiedenen Erscheinungsformen stammt.

10. Verwendung nach einem der Ansprüche 6 oder 8, **dadurch gekennzeichnet, daß** das gasförmige Arzneimittel gebrauchsfähig ist.
